# EUROPÄISCHE PATENTSCHRIFT

(11) **EP 1 157 013 B1**
(45) Veröffentlichungstag und Bekanntmachung des Hinweises auf die Patenterteilung: **08.12.2004**
(21) Anmeldenummer: 00906286.0
(22) Anmeldetag: 05.02.2000
(51) Int. Cl.: C07D 277/60, C07D 513/04, A61K 31/428, A61K 31/429, A61P 3/04

(54) **POLYCYCLISCHE THIAZOL-2-YLIDEN AMINE, VERFAHREN ZU IHRER HERSTELLUNG UND IHRE VERWENDUNG ALS ARZNEIMITTEL**
POLYCYCLIC THIAZOLE-2-YLIDES AMINES, METHOD FOR THE PRODUCTION THEREOF AND THEIR UTILIZATION AS MEDICAMENTS
THIAZOLIDIN-2-YLIDENAMINES POLYCYCLIQUES, LEUR PROCEDE DE PRODUCTION, ET LEUR UTILISATION COMME PRODUIT PHARMACEUTIQUE

(30) Priorität: 26.02.1999 DE 19908536
(43) Veröffentlichungstag der Anmeldung: 28.11.2001
(73) Patentinhaber: Aventis Pharma Deutschland GmbH, 65929 Frankfurt am Main (DE)
(72) Erfinder: JÄHNE, Gerhard, D-65929 Frankfurt am Main (DE); GEISEN, Karl, D-60318 Frankfurt (DE); LANG, Hans-Jochen, D-65719 Hofheim (DE); BICKEL, Martin, D-61348 Bad Homburg (DE)
(86) Internationale Anmeldenummer: PCT/EP2000/000926
(87) Internationale Veröffentlichungsnummer: WO 2000/051996

(56) Entgegenhaltungen:
- US-A- 3 507 868
- US-A- 4 174 397
- US-A- 5 869 492
- ARYA, V. P. ET AL: "Synthesis of new heterocycles. III. Syntheses of certain novel condensed imidazo[2,1-b]thiazoles and thiazolo[3,2-.alpha.]pyrimidines" INDIAN J. CHEM. (1971), 9(11), 1204-8 , XP000887076
- DESTEVENS, G. ET AL: "Investigations in Heterocycles. II. Unsymmetrical Ureas, Thioureas and Related Thiazolines" JOURNAL OF ORGANIC CHEMISTRY., Bd. 23, 1958, Seiten 114-116, XP000887058 AMERICAN CHEMICAL SOCIETY. EASTON., US ISSN: 0022-3263

## Beschreibung

Die Erfindung betrifft polycyclische Thiazol-2-yliden Amine sowie deren physiologisch verträgliche Salze und physiologisch funktionelle Derivate.

In US 3,507,868 sind bereits im Ring A unsubstituierte tetracyclische Imidazo[2,1-b]thiazole und Thiazolo[3,2-a]pyrimidine mit anorektischer Wirkung beschrieben.

In US 4,174,397 sind strukturähnliche Thiazolidin derivate mit anorektischer Wirkung beschrieben.

Der Erfindung lag die Aufgabe zugrunde, weitere Verbindungen zur Verfügung zu stellen, die eine therapeutisch verwertbare anorektische Wirkung entfalten. In diesem Zusammenhang bestand die Aufgabe insbesondere auch darin, Verbindungen zu finden, bei denen die anorektische Wirkung gegenüber den Verbindungen aus US 3,507,868 erhöht ist und bei denen weniger Nebenwirkungen auftreten.

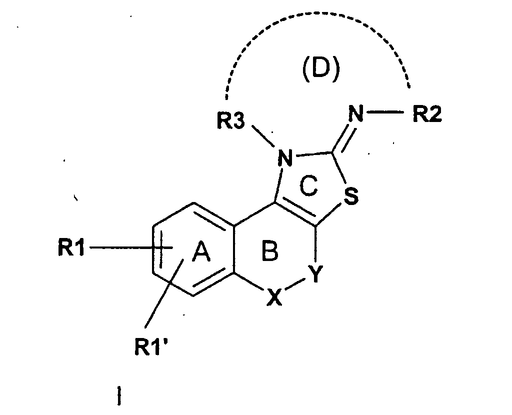

Die Erfindung betrifft daher Verbindungen der Formel I,
worin bedeuten
- Y: eine direkte Bindung,
- X: CH₂, CH-Phenyl,
- R1: F, Cl, Br, J, O-(C₂-C₆)-Alkyl, wobei in den Alkylresten ein, mehrere, oder alle Wasserstoff(e) durch Fluor ersetzt sein können, ersetzt sein kann; O-(CH₂)ₙ-Phenyl, wobei n = 0 - 6 sein kann, wobei der Phenyl ring, jeweils ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂;
- R1': H, F, Cl, Br, J, O-(C₂-C₆)-Alkyl wobei in den Alkylresten ein, mehrere, oder alle Wasserstoff(e) durch Fluor ersetzt sein können, O-(CH₂)ₙ-Phenyl, wobei n = 0 - 6 sein kann, wobei der Phenyl ring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- R2: (C₁-C₆)-Alkyl, (CH2)ₙ-COOH, wobei n = 1-4 sein kann; (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann;
- R3: (C₁-C₆)-Alkyl, (CH2)ₙ-COOH, wobei n = 1-4 sein kann; (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, oder O-(C₁-C₆)-Alkyl substituiert sein kann;
oder
- R2 und R3: bilden gemeinsam eine -CH₂-CH₂-, -CH₂-C(CH₃)₂-, -CH₂-CH₂-CH₂-, oder -CH₂-CH₂-CH₂-CH₂- Gruppe;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.
Die Alkyl-, Alkenyl- und Alkinylreste in den Substituenten R1, R1', R2 und R3 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorid verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion sind nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Ein Verfahren zur Herstellung der Verbindungen der allgemeinen Formel ist dadurch gekennzeichnet, daß man die Verbindungen der Formel I so gewinnt, daß gemäß dem folgenden Reaktionsschema vorgegangen wird:

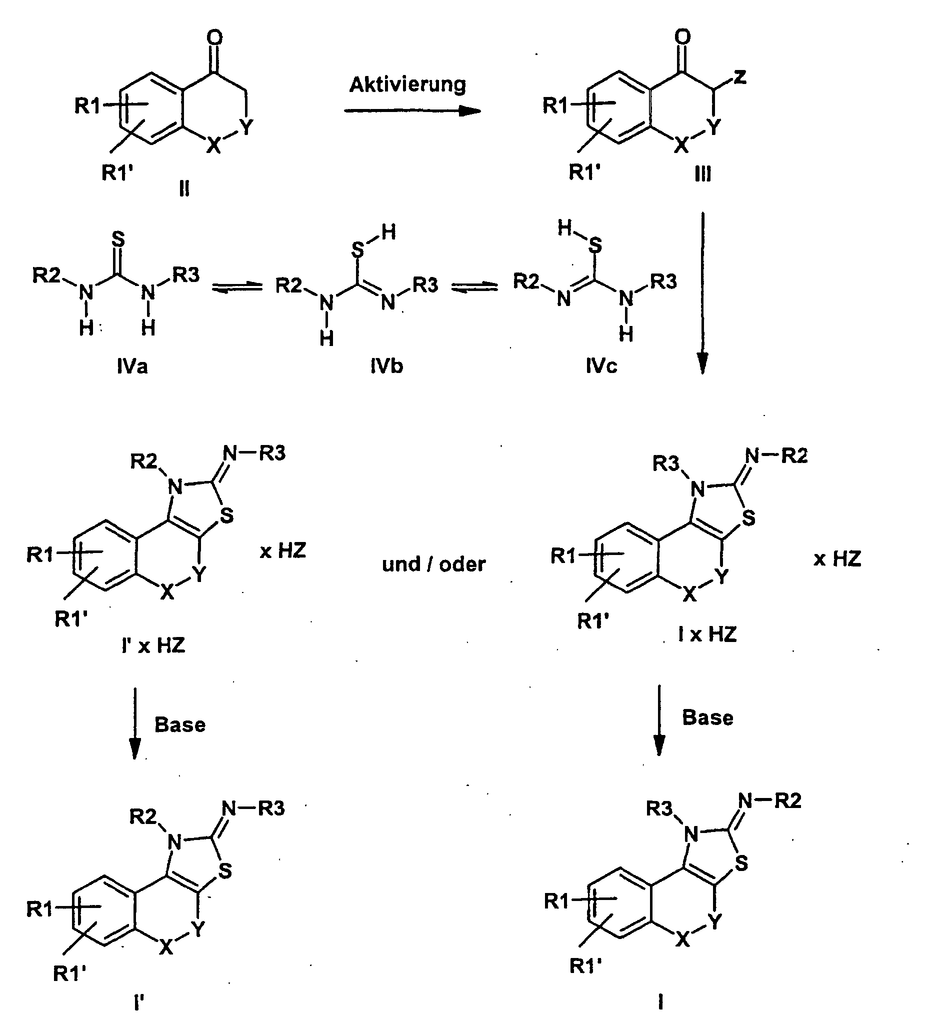

Dazu werden Verbindungen der allgemeinen Formel II, 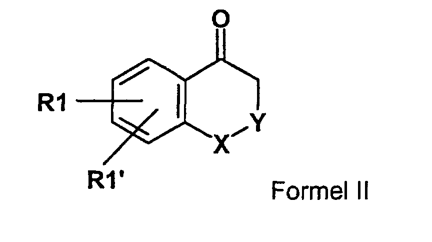 worin R1, R1', X und Y die angegebene Bedeutung besitzen, aktiviert und in eine Verbindung der Formel III überführt, worin Z für den Rest eines aktivierten Esters einer anorganischen oder organischen Säure steht.
Die Verbindungen der Formel III werden weiter mit Thioharnstoffen der Formel IVa, 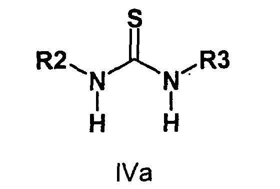 die mit Verbindungen der Formeln IVb und IVc in einem tautomeren Gleichgewicht stehen können und worin R2 und R3 die angegebenen Bedeutungen besitzen, zu Verbindungen der Formel I x HZ bzw. I' x HZ umgesetzt, wobei gegebenenfalls die Verbindungen der allgemeinen Formel I x HZ bzw. I' xHZ mit organischen oder anorganischen Basen in ihre freie Form der Formel I bzw I' überführt werden. Diese wiederum können mit Hilfe einer anorganischen oder organischen Säure in ein anderes Säureadditionssalz überführt werden.

Als anorganische Säuren kommen beispielsweise in Betracht:
Halogenwasserstoffsäuren wie Chlorwasserstoffsäure und Bromwasserstoffsäure, sowie Schwefelsäure, Phosphorsäure und Amidosulfonsäure.

Als organische Säuren seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die oben beschriebene Verfahrensweise wird vorteilhaft so ausgeführt, daß man die Verbindungen III mit den Thioharnstoffen IVa im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn das jeweilige Thioamid einen möglichst tiefen Schmelzpunkt hat. Die Reaktion verläuft nur wenig exotherm und kann zwischen - 10°C und 150°C, bevorzugt zwischen 30°C und 130°C, durchgeführt werden. Als besonders günstig erwies sich in der Regel ein Temperaturbereich zwischen 70°C und 110°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Häufig scheiden sich die Verbindungen I und I' in Form ihrer Säureadditionssalze I x HZ und I' x HZ im Verlauf der Reaktion schwerlöslich ab, zwechmäßig wird nachträglich noch ein geeignetes Fällungsmittel zugesetzt. Als solches verwendet man z.B. Kohlenwasserstoffe wie Benzol, Toluol, Cyclohexan oder Heptan oder Tetrachlormethan; insbesondere erwiesen sich Essigsäurealkylester wie Essigsäureethylester oder Essigsäure-n-butylester oder Dialkylether wie Diethylether, Diisopropylether, Di-n-butylether oder tert.Butylmethylether als besonders geeignet. Bleibt nach Ende der Reaktion das Reaktionsgemisch in Lösung, so kann man die Salze der Verbindungen I oder I', ggf. nach Konzentrierung der Reaktionslösung, mit einem der genannten Fällungsmittel ausfällen. Ferner kann man die Lösung des Reaktionsgemisches auch vorteilhaft in die Lösung eines der genannten Fällungsmittel unter Rühren einfiltrieren. Da die Reaktion der Verbindungen III mit den Thioharnstoffen IVa praktisch quantitativ abläuft, sind die erhaltenen Rohprodukte meistens bereits analytisch rein. Die Aufarbeitung des Reaktionsgemisches kann auch so durchgeführt werden, daß die Reaktionsmischung unter Zusatz einer organischen Base, wie z.B. Triethylamin oder Diisobutylamin oder Ammoniak oder Morpholin oder Piperidin oder 1,8-Diazabicyclo[5.4.0]undec-7-en alkalisch gestellt wird, und das Reaktionsrohprodukt nach Konzentrierung chromatographisch, z.B. über eine Kieselgelsäule, gereinigt wird. Als geeignete Elutionsmedien dafür erweisen sich z.B. Gemische von Essigsäureethylester mit Methanol, Gemische von Dichlormethan mit Methanol, Gemische von Toluol mit Methanol oder Essigsäureethylester oder Gemische von Essigsäurethylester mit Kohlenwasserstoffen wie Heptan. Erfolgt die Reinigung des Rohproduktes auf die zuletzt beschriebene Weise, kann aus der so gewonnenen reinen Base der Formel I oder I' ein Säureadditionsprodukt so gewonnen werden, daß man die Base in einem organischen protischen Lösemittel wie Methanol, Ethanol, Propanol oder Isopropanol oder in einem organischen aprotischen Lösemittel wie Essigsäureethylester, Diethylether, Diisopropylether, tert.Butylmethylether, Dioxan, Tetrahydrofuran, Aceton oder Butan-2-on löst oder suspendiert und diese Mischung anschließend mit einer wenigstens äquimolaren Menge einer anorganischen Säure wie z.B. Chlorwasserstoffsäure, gelöst in einem inerten Lösemittel wie z.B. Diethylether oder Ethanol, oder einer anderen der weiter oben genannten anorganischen oder organischen Säuren versetzt.

Die Verbindungen der Formel I und I' können aus einem inerten, geeigneten Lösemittel wie z.B. Aceton, Butan-2-on, Acetonitril, Nitromethan umkristallisiert werden. Besonders vorteilhaft ist aber die Umfällung aus einem Lösemittel wie z.B. Dimethylformamid, Dimethylacetamid, Nitromethan, Acetonitril, vorzugsweise Methanol oder Ethanol.

Die Reaktion der Verbindungen der Formel III mit den Thioharnstoffen der Formel IVa kann auch so durchgeführt werden, daß man zur Reaktionsmischung eine wenigstens äquimolare Menge einer Base, wie z.B. Triethylamin, zufügt und die so erhaltenen Verbindungen I oder I' anschließend gegebenenfalls in ihre Säureadditionsprodukte überführt.

In den Verbindungen der Formel III kommen als Rest eines aktivierten Esters Z beispielsweise in Frage: Cl, Br, J, O-C(O)-(C₆H₄)-4-NO₂, O-SO₂-CH₃, O-SO₂-CF₃, O-SO₂-(C₆H₄)-4-CH₃, O-SO₂-C₆H₅.

Die Säureadditionsprodukte I x HZ und I' x HZ können durch Behandlung mit Basen zu den Verbindungen der allgemeinen Formel I und I' umgesetzt werden. Als Basen kommen beispielsweise Lösungen anorganischer Hydroxide, wie Lithium-, Natrium-, Kalium-, Calcium- oder Bariumhydroxid, Carbonate oder Hydrogencarbonate, wie Natrium- oder Kaliumcarbonat, Natrium- oder Kaliumhydrogencarbonat, Ammoniak und Amine, wie Triethylamin, Diisopropylamin, Dicyclohexylamin, Piperidin, Morpholin, Methyldicyclohexylamin in Frage.

Thioharnstoffe der allgemeinen Formel IVa sind entweder kommerziell erhältlich oder können nach literaturbekannten Methoden hergestellt werden.

Erfindungsgemäß wurden außer den in den Ausführungsbeispielen beschriebenen Derivaten auch die in der folgenden Tabelle 1 zusammengestellten Verbindungen der allgemeinen Formel I bzw. deren Säureadditionsprodukte erhalten:

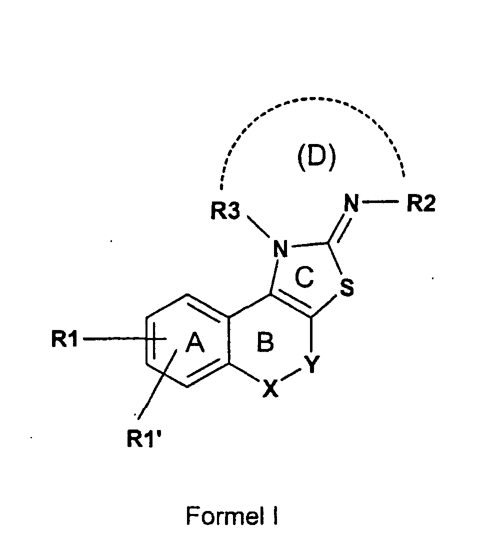

**Tabelle 1:**

| Beispiele | | | | | | | |
|---|---|---|---|---|---|---|---|
| Beispiel | R₁; R₁' | R₂ | R₃ | Y | X | Salz | Fp. [°C] |
| 3 | 6-Cl | CH₃ | CH₃ | - | CH₂ | HBr | 278 |
| 4 | 6-Cl | CH₃ | CH₃ | - | CH₂ | HCl | 281 |
| 5 | 6-Cl | CH₂-CH₂ | | - | CH₂ | HCl | 258 |
| 6 | 6-Cl | CH₂-COOH | CH₃ | - | CH₂ | HBr | 256 |
| 7 | 6-Cl | CH₂-COOH | C₆H₅ | - | CH₂ | HBr | 243 |
| 11 | 7-Cl | CH₃ | CH₃ | - | CH₂ | HCl | 250 |
| 12 | 5-Cl | CH₃ | CH₃ | - | CH₂ | HCl | 249 |
| 13 | 6-F | CH₃ | CH₃ | - | CH₂ | HCl | 250 |
| 14 | 6-Cl | CH₃ | CH₃ | - | CH₂ | - | 171 |
| 15 | 6-(O-C₆H₄)-4-Cl | CH₃ | CH₃ | - | CH₂ | HCl | 251 |
| 16 | 6-O-CH₂-CF₃ | CH₃ | CH₃ | - | CH₂ | HCl | 276 |
| 17 | 6-O-CH₂-CF₂-CF₃ | CH₃ | CH₃ | - | CH₂ | HCl | 240 |
| 20 | 6-(O-C₆-H₄)-3-CH₃) | CH₃ | CH₃ | - | CH₂ | HCl | 229 |
| 21 | 6-O-CH₂-CF₂-CF₂-CF₃ | CH₂-CH₂-CH₂ | | - | CH₂ | HCl | 271 |
| 22 | 6-O-CH₂-CF₂-CF₂-CF₃ | CH₃ | CH₃ | - | CH₂ | HCl | 251 |
| 23 | 6-Cl | C₆H₅ | C₆H₅ | - | CH₂ | HBr | 213 |
| 24 | 6-Cl | (C₆H₄)-4-Cl | (C₆H₄)-4-Cl | - | CH₂ | - | 235 |
| 25 | 6-Cl | (C₆H₄)-4-OCH₃ | (C₆H₄)-4-OCH₃ | - | CH₂ | HBr | 243 |
| 26 | 6-(O-C₆H₅) | CH₂-CH₂ | | - | CH₂ | HBr | 243 |
| 27 | 6-(O-C₆H₅) | CH₃ | CH₃ | - | CH₂ | HBr | 205 |
| 28 | 6-(O-C₆H₄-3-CH₃) | CH₂-CH₂ | | - | CH₂ | HBr | 250 |
| 30 | 6-Cl | C₆H₅ | CH₂-CH₂-O-CH₃ | - | CH₂ | HBr | 231 |
| 31 | 6-Cl | C₆H₅ | CH₂-CH₂-C₆H₅ | - | CH₂ | HBr | 266 |
| 32 | 6-Cl | CH₃ | C₆H₄-4-C(O)O-CH₂-CH₃ | - | CH₂ | HBr | 223 |

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Die Verbindungen eignen sich zur Prophylaxe sowie insbesondere zur Behandlung von Obesitas. Die Verbindungen eignen sich weiterhin zur Prophylaxe sowie zur Behandlung von Typ II Diabetes.

### Biologisches Prüfmodell:

Die Prüfung der anorektischen Wirkung erfolgte an männlichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

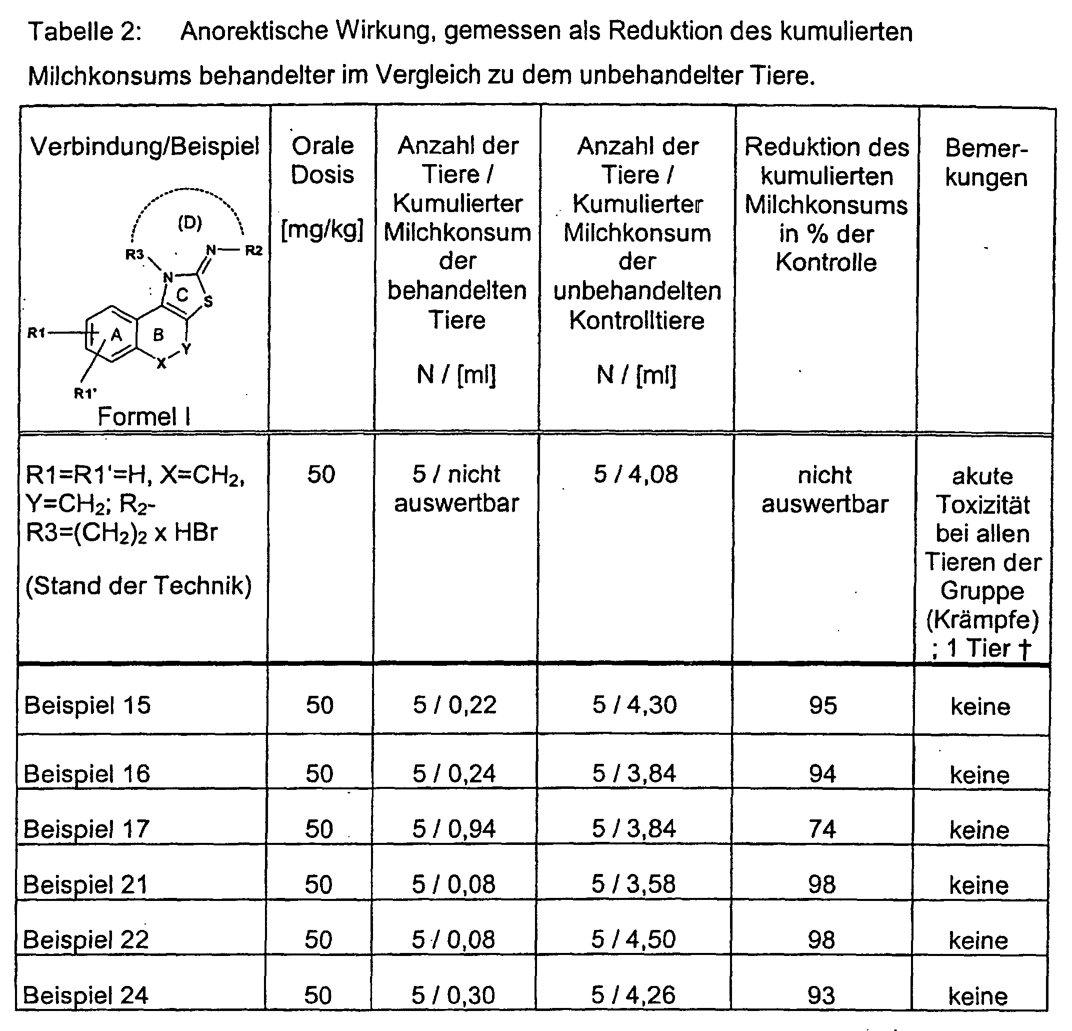

Aus der Tabelle ist abzulesen, daß die Verbindungen der Formel I eine gute anorektische Wirkung zeigen. Bei den Versuchstieren wurden keine Nebenwirkungen beobachtet.

Die nachfolgend aufgeführten Beispiele dienen zur Erläuterung der Erfindung, ohne diese jedoch einzuschränken. Die angegebenen Zersetzungspunkte sind nicht korrigiert und generell von der Aufheizgeschwindigkeit abhängig.

### Ausführungsbeispiel 1:

### (6-Chlor-3-methyl-3,8-dihydro-indeno[1,2-d]thiazol-2-yliden)-methyl-amin Hydrobromid: (Verbindung des Beispiels 3):

a) 6-Chlor-3-methyl-2-methylimino-2,3,8,8a-tetrahydro-indeno[1,2-d]thiazol-3aol Hydrobromid:
   2,47 g (10 mMol) 2-Brom-5-chlor-indan-1-on werden in 30 ml Aceton gelöst und bei Raumtemperatur mit einer Lösung von 1,05 g (10 mMol) N,N'-Dimethylthioharnstoff in 10 ml Aceton versetzt und 5 h bei Raumtemperatur gerührt. Der Niederschlag wird abgesaugt und im Vakuum getrocknet. Nach Kristallisation aus Methanol/Diethylether erhält man das Hydrobromid des 6-Chlor-3-methyl-2-methylimino-2,3,8,8a-tetrahydro-indeno[1,2-d]thiazol-3a-ols mit dem Schmelzpunkt 181-183°C.
b) (6-Chlor-3-methyl-3,8-dihydro-indeno[1,2-d]thiazol-2-yliden)-methyl-amin:
   3,05 g (10mMol) 6-Chlor-3-methyl-2-methylimino-2,3,8,8a-tetrahydroindeno[1,2-d]thiazol-3a-ol Hydrobromid werden in 20 ml Eisessig suspendiert und 4 h unter Rückfluß gerührt. Man läßt auf Raumtemperatur abkühlen und saugt den Niederschlag ab. Man erhält das Hydrobromid des (6-Chlor-3-methyl-3,8-dihydro-indeno[1,2-d]thiazol-2-yliden)methyl-amins mit dem Schmelzpunkt 278°C.

### Ausführungsbeispiel 4:

### [6-(4-Chlor-phenoxy)-3-methyl-3,8-dihydro-indeno[1,2-d]thiazol-2-ylidene]-methylamin Hydrochlorid (Verbindung des Beispiels 15):

6-(4-Chlorphenoxy)-3-methyl-2-methylamino-8,8a-dihydro-indeno[1,2-d]thiazol-3a-ol Hydrochlorid werden in 15 ml Eisessig für 4 Stunden unter Rückfluß gekocht, das Lösungsmittel unter vermindertem Druck abdestilliert und der Rückstand unter Diisopropylether zur Kristallisation gebracht. Schmp. 250-252°C

### Ausführungsbeispiel 6:

### 7-Phenoxy-4H,9H-2,3-dihydroimidazo[2,1-b]indeno[1,2-d]thiazol Hydrobromid (Verbindung des Beispiels 24):

0,73 g des entsprechenden Hydrobromides des 9aH,4aH,4a-Hydroxy-Derivates werden in 20 ml Eisessig für 2 Stunden auf 100°C erhitzt, das Lösungsmittel abdestilliert und der Rückstand unter Diisopropylether zur Kristallisation gebracht. Schmp. 235°C.

## Patentansprüche

1. Verbindungen der Formel I, 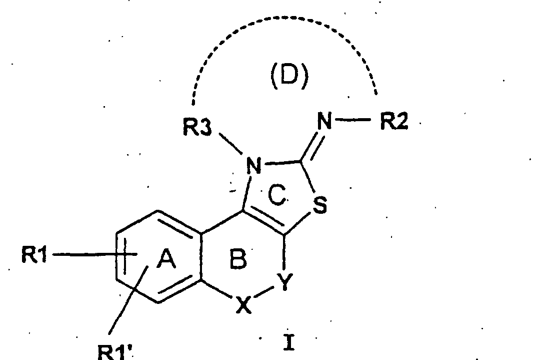 worin bedeuten
Y eine direkte Bindung,
X CH₂, CH-Phenyl,
R1 F, Cl, Br, J, O-(C₂-C₆)-Alkyl, wobei in den Alkylresten ein, mehrere, oder alle Wasserstoff(e) durch Fluor ersetzt sein können, O-(CH₂)ₙ-Phenyl, wobei n = 0 - 6 sein kann, wobei der Phenyl ring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂;
R1' H, F, Cl, Br, J, O-(C₂-C₆)-Alkyl, wobei in den Alkylresten ein, mehrere, oder alle Wasserstoff(e) durch Fluor ersetzt sein können, O-(CH₂)ₙ-Phenyl, wobei n = 0 - 6 sein kann, wobei der Phenyl ring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R2 (C₁-C₆)-Alkyl, (CH2)ₙ-COOH, wobei n = 1-4 sein kann; (C₃-C₆)-Cycloalkyl, (CH₂)n-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C1-C₆)-Alkyl substituiert sein kann;
R3 (C₁-C₆)-Alkyl, (CH₂)n-COOH, wobei n = 1-4 sein kann; (C₃-C₆)-Cycloalkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, oder O-(C₁-C₆)-Alkyl substituiert sein kann;
oder
R2 und R3 bilden gemeinsam eine -CH₂-CH₂-, -CH₂-C(CH₃)₂-, -CH₂-CH₂-CH₂-, oder -CH₂-CH₂-CH₂-CH₂- Gruppe;
sowie deren physiologisch verträgliche Salze.

2. Arzneimittel enthaltend eine oder mehrere der Verbindungen gemäß Anspruch 1.

3. Arzneimittel enthaltend eine oder mehrere der Verbindungen gemäß Anspruch 1 und ein oder mehrere anorektische Wirkstoffe.

4. Verbindungen gemäß Anspruch 1 zur Anwendung als Medikament zur Prophylaxe oder Behandlung der Obesitas.

5. Verbindungen gemäß Anspruch 1 zur Anwendung als Medikament zur Prophylaxe oder Behandlung des Typ II Diabetes.

6. Verbindungen gemäß Anspruch 1 in Kombination mit mindestens einem weiteren anorektischen Wirkstoff zur Anwendung als Medikament zur Prophylaxe oder Behandlung der Obesitas.

7. Verbindungen gemäß Anspruch 1 in Kombination mit mindestens einem weiteren anorektischen Wirkstoff zur Anwendung als Medikament zur Prophylaxe oder Behandlung der des Typ II Diabetes.

8. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

9. Verwendung der Verbindungen gemäß Anspruch 1 zur Herstellung eines Medikaments zur Prophylaxe oder Behandlung der Obesitas.

10. Verwendung der Verbindungen gemäß Anspruch 1 zur Herstellung eines Medikaments zur Prophylaxe oder Behandlung des Typ II Diabetes.

## Claims

1. A compound of the formula I 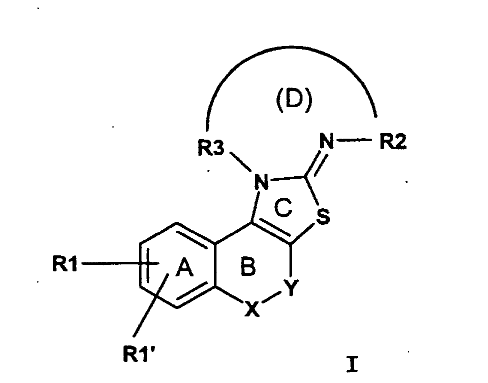 in which
Y is a direct linkage,
X is CH₂, CH-phenyl,
R1 is F, Cl, Br, I, O-(C₂-C₆)-alkyl, where one, more than one or all hydrogen(s) in the alkyl radicals can be replaced by fluorine, or O-(CH₂)ₙ-phenyl, where n can be 0-6, it being possible for the phenyl ring to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl or CONH₂;
R1' is H, F, Cl, Br, I, O-(C₂-C₆)-alkyl, where one, more than one or all hydrogen(s) in the alkyl radicals can be replaced by fluorine, or O-(CH₂)ₙ-phenyl, where n can be 0-6, it being possible for the phenyl ring to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R2 is (C₁-C₆)-alkyl, (CH₂)ₙ-COOH, where n can be 1-4; (C₃-C₆)-cycloalkyl, (CH₂)ₙ-phenyl, (CH₂)ₙ-thienyl, (CH₂)ₙ-pyridyl, (CH₂)ₙ-furyl, where n can be 0-5 and in which phenyl, thienyl, pyridyl, furyl can each be substituted up to twice by Cl, F, CN, CF₃, (C₁-C₃)-alkyl, OH or O-(C₁-C₆)-alkyl;
R3 is (C₁-C₆)-alkyl, (CH₂)ₙ-COOH, where n can be 1-4; (C₃-C₆)-cycloalkyl, (CH₂)ₙ-phenyl, (CH₂)ₙ-thienyl, (CH₂)ₙ-pyridyl, (CH₂)ₙ-furyl, where n can be 0-5 and in which phenyl, thienyl, pyridyl, furyl can each be substituted up to twice by Cl, F, CN, CF₃, (C₁-C₃)-alkyl, OH or O-(C₁-C₆)-alkyl;
or
R2 and R3 together form a -CH₂-CH₂-, -CH₂-C(CH₃)₂-, -CH₂-CH₂-CH₂- or -CH₂-CH₂-CH₂-CH₂- group;
and their physiologically tolerated salts.

2. A medicament comprising one or more compounds as claimed in claim 1.

3. A medicament comprising one or more compounds as claimed in claim 1 and one or more anorectic active ingredients.

4. A compound as claimed in claim 1 for use as medicine for the prophylaxis or treatment of obesity.

5. A compound as claimed in claim 1 for use as medicine for the prophylaxis or treatment of type II diabetes.

6. A compound as claimed in claim 1 in combination with at least one other anorectic active ingredient for use as medicine for the prophylaxis or treatment of obesity.

7. A compound as claimed in claim 1 in combination with at least one other anorectic active ingredient for use as medicine for the prophylaxis or treatment of type II diabetes.

8. A process for producing a medicament comprising one or more compounds as claimed in claim 1, which comprises mixing the active ingredient with a pharmaceutically suitable carrier, and converting this mixture into a form suitable for administration.

9. The use of the compounds as claimed in claim 1 for producing a medicine for the prophylaxis or treatment of obesity.

10. The use of the compounds as claimed in claim 1 for producing a medicine for the prophylaxis or treatment of type II diabetes.

## Revendications

1. Composés de formule I, 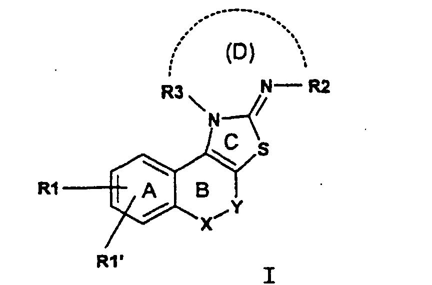 dans laquelle
Y représente une liaison directe,
X représente CH₂, CH-phényle,
R1 représente F, Cl, Br, I, O-(C₂-C₆)alkyle, où dans les radicaux alkyle, un, plusieurs ou la totalité des hydrogènes peuvent être remplacés par un fluor, O-(CH₂)ₙ-phényle, dans lequel n peut valoir de 0 à 6, où le noyau phényle peut être mono- à tri-substitué par F, Cl, Br, I, OH, CF₃, NO₂, CN, OCF₃, O-(C₁-C₆)alkyle, un alkyle en C₁-C₆, NH₂, NH(C₁-C₆)alkyle, N((C₁-C₆)alkyle)₂, SO₂-CH₃, COOH, COO- (C₁-C₆) alkyle ou CONH₂ ;
R1' représente H, F, Cl, Br, I, O-(C₂-C₆) alkyle, où dans les radicaux alkyle, un, plusieurs ou la totalité des hydrogènes peuvent être remplacés par un fluor, O-(CH₂)ₙ-phényle, dans lequel n peut valoir de 0 à 6, où le noyau phényle peut être mono- à tri-substitué par F, Cl, Br, I, OH, CF₃, NO₂, CN, OCF₃, O-(C₁-C₆)alkyle, un alkyle en C₁-C₆, NH₂, NH(C₁-C₆)alkyle, N((C₁-C₆) alkyle)₂, SO₂-CH₃, COOH, COO-(C₁-C₆)alkyle ou CONH₂ ;
R2 représente un alkyle en C₁-C₆, (CH₂)ₙ-COOH, dans lequel n peut valoir de 1 à 4 ; un cycloalkyle en C₃-C₆, (CH₂)ₙ-phényle, (CH₂)ₙ-thiényle, (CH₂)ₙ-pyridyle, (CH₂)ₙ-furyle, dans lesquels n peut valoir de 0 à 5 et dans lesquels le phényle, le thiényle, le pyridyle, le furyle peut, dans chaque cas, être jusqu'à bi-substitué par Cl, F, CN, CF₃, un alkyle en C₁-C₃, OH ou O-(C₁-C₆) alkyle ;
R3 représente un alkyle en C₁-C₆, (CH₂)ₙ-COOH, dans lequel n peut valoir de 1 à 4 ; un cycloalkyle en C₃-C₆, (CH₂)ₙ-phényle, (CH₂)ₙ-thiényle, (CH₂)ₙ-pyridyle, (CH₂)ₙ-furyle, dans lesquels n peut valoir de 0 à 5 et dans lesquels le phényle, le thiényle, le pyridyle, le furyle peut, dans chaque cas, être jusqu'à bi-substitué par Cl, F, CN, CF₃, un alkyle en C₁-C₃, OH ou O-(C₁-C₆) alkyle ;
ou
R2 et R3 forment conjointement un groupe -CH₂-CH₂-, -CH₂-C(CH₃)₂-, -CH₂-CH₂-CH₂- ou -CH₂-CH₂-CH₂-CH₂- ; ainsi que leurs sels physiologiquement acceptables.

2. Médicament comprenant un ou plusieurs des composés selon la revendication 1.

3. Médicament comprenant un ou plusieurs des composés selon la revendication 1 et un ou plusieurs ingrédients actifs anorexiants.

4. Composés selon la revendication 1, destinés à être utilisés en tant que médicament pour la prophylaxie ou le traitement de l'obésité.

5. Composés selon la revendication 1, destinés à être utilisés en tant que médicament pour la prophylaxie ou le traitement du diabète du type II.

6. Composés selon la revendication 1, en combinaison avec au moins un autre ingrédient actif anorexiant, destinés à être utilisés en tant que médicament pour la prophylaxie ou le traitement de l'obésité.

7. Composés selon la revendication 1, en combinaison avec au moins un autre ingrédient actif anorexiant, destinés à être utilisés en tant que médicament pour la prophylaxie ou le traitement du diabète du type II.

8. Procédé de production d'un médicament comprenant un ou plusieurs composés selon la revendication 1, **caractérisé en ce que** l'ingrédient actif est mélangé avec un support pharmaceutiquement acceptable, et ce mélange est transformé en une forme acceptable pour l'administration.

9. Utilisation des composés selon la revendication 1 pour la production d'un médicament pour la prophylaxie ou le traitement de l'obésité.

10. Utilisation des composés selon la revendication 1 pour la production d'un médicament pour la prophylaxie ou le traitement du diabète du type II.
